# EUROPEAN PATENT SPECIFICATION

(11) **EP 2 743 261 B1**
(45) Date of publication and mention of the grant of the patent: **29.11.2017**
(21) Application number: 12822810.3
(22) Date of filing: 24.04.2012
(51) Int. Cl.: C07D 207/273

(54) **PREPARATION METHOD OF CRYSTAL FORM I OF THE RACEMATE OF 4-HYDROXY-2-OXO-1-PYRROLIDINE-ACETAMIDE**
HERSTELLUNGSVERFAHREN DER KRISTALLFORM I DES 4-HYDROXY-2-OXO-1-PYRROLIDIN-ACETAMID-RACEMATS
PROCÉDÉ DE PRÉPARATION DE LA FORME CRISTALLINE I DU RACÉMATE DE 4-HYDROXY-2-OXO-1-PYRROLIDINE-ACÉTAMIDE

(30) Priority: 11.08.2011 CN 201110230079
(43) Date of publication of application: 18.06.2014
(73) Proprietor: CHONGQING RUZER PHARMACEUTICAL CO., LTD., Yubei District Chongqing 401120 (CN)
(72) Inventor: YE, Lei, Yubei Chongqing 401120 (CN)
(74) Representative: Oudin, Stéphane
(86) International application number: PCT/CN2012/074573
(87) International publication number: WO 2013/020390

(56) References cited:
- EP-A1- 0 223 328
- CN-A- 1 948 285
- CN-A- 101 121 688
- CN-A- 101 693 685
- CN-A- 101 885 697
- CN-A- 102 249 977
- US-A- 4 797 496
- G. PIFFERI AND M. PINZA: "Cyclic GABA-GABOB analogues I - Synthesis of new 4-hydroxy-2-pyrrolidinone derivatives", FARMACO, EDIZIONE SCIENTIFICA, vol. 32, no. 8, 1977, pages 602-613, XP001008299, ISSN: 0430-0920
- GUILIANO BANDOLI ET AL: "Solid-state structure and conformation of the nootropic agent 4-hydroxy-2-oxo-1-pyrrolidineacetamide: X-ray and theoretical self-consistent field molecular orbital (SCF-MO) studies", CHEMICAL & PHARMACEUTICAL BULLETIN, vol. 33, no. 10, 1985, pages 4395-4401, XP055153439, ISSN: 0009-2363
- DAVID D. P. LAFFAN ET AL: "An efficient synthesis of racemic 4-hydroxy-2-oxo-1-pyrrolidineacetamide (Oxiracetam) using tetramic-acid intermediates", HELVETICA CHIMICA ACTA, vol. 75, no. 3, 1992, pages 892-900, XP009028970, ISSN: 0018-019X, DOI: 10.1002/HLCA.19920750323

## Description

### Field of the Invention

The present invention relates to a preparation method of crystal form I of the racemate of 4-hydroxy-2-oxo-1-pyrrolidine acetamide.

### Description of the Prior art

4-hydroxy-2-oxo-1-pyrrolidine acetamide (oxiracetam), as a new generation of cerebral metabolism improving agent, a derivative of Pyrrolidinone (ring GABOB) and an analog of Piracetam, can promote the synthesis of phosphorylcholine and phosphoryl ethanolamine and promote cerebral metabolism, shows stimulatory effects to specific central nervous pathway through blood-brain barrier, improves intelligence and memory, shows good therapeutic effects on cerebrovascular disease, brain injury, brain tumor (after operation), intracranial infection, dementia, cerebral degenerative disease and the like, and is suitable for memory and intelligence impairment caused by mild to moderate vascular dementia, senile dementia, brain injury and other diseases. 4-hydroxy-2-oxo-1-pyrrolidine acetamide, was first synthesized by Italian ISF company in 1974 and came into the market in 1987, which can promote the synthesis of phosphorylcholine and phosphoryl ethanolamine, promote cerebral metabolism, has better effects on memory and especially the focus of thinking than Piracetam and has low toxicity. 4-hydroxy-2-oxo-1-pyrrolidine acetamide is a racemate which consists of two isomers (S)-4-hydroxy-2-oxo-1-pyrrolidine acetamide and (R)-4-hydroxy-2-oxo-1-pyrrolidine acetamide, the chemical name of which is 4-hydroxy-2-oxo-1-pyrrolidine acetamide, and the chemical structure is shown below:

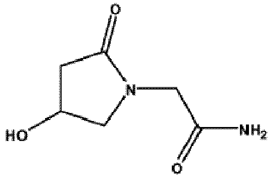

The synthetic methods of 4-hydroxy-2-oxo-1-pyrrolidine acetamide, the preparation methods and products of injection fomulations, and dispersible tablets and preparation methods thereof have been reported by literatures.

### Summary of the Invention

It is an objective of the present invention to provide a crystal form I of the racemate of 4-hydroxy-2-oxo-1-pyrrolidine acetamide" which is in high purity and has good medicinal therapeutic effects.

The crystal form of the racemate of 4-hydroxy-2-oxo-1-pyrrolidine acetamide in the present invention is named as the crystal form I of the racemate of 4-hydroxy-2-oxo-1-pyrrolidine acetamide by the inventors of the present application.

The objective of the present invention is more specifically to provide a preparation method of the crystal form I of the racemate of 4-hydroxy-2-oxo-1-pyrrolidine acetamide. The crystal form I of the racemate of 4-hydroxy-2-oxo-1-pyrrolidine acetamide obtained by this method contains low content of impurities and has high purity.

The crystal form I of the racemate of 4-hydroxy-2-oxo-1-pyrrolidine acetamide is characterized by having diffraction peaks appear at the following 2θ diffraction angles: 12.011, 15.318, 17.407, 19.633, 21.228, 22.052, 24.577, 25.223, 27.647, 28.161, 29.109, 30.805, 31.276, 31.766, 32.77, 33.477, 35.252, 35.645, 36.236, 37.379, 39.56, 40.489, 41.256, 41.948, 43.443 and 44.628.

The crystal form I of the racemate of 4-hydroxy-2-oxo-1-pyrrolidine acetamide is characterized by a X-ray powder diffraction data expressed in terms of d (Å)-values and relative intensity percentages I (%) as shown below:

| d value | I value | d value | I value |
|---|---|---|---|
| 7.3623 | 3.6 | 5.7794 | 11.6 |
| 5.0905 | 34.7 | 4.5179 | 51.2 |
| 4.1820 | 100.0 | 4.0275 | 5.8 |
| 3.6192 | 49.1 | 3.5278 | 6.4 |
| 3.2238 | 15.6 | 3.1661 | 13.2 |
| 3.0652 | 10.4 | 2.9002 | 1.3 |
| 2.8576 | 1.4 | 2.8146 | 5.6 |
| 2.7306 | 12.3 | 2.6746 | 5.6 |
| 2.5438 | 5.0 | 2.5167 | 8.4 |
| 2.4770 | 7.9 | 2.4038 | 1.9 |
| 2.2762 | 2.3 | 2.2261 | 3.7 |
| 2.1865 | 1.5 | 2.1520 | 2.6 |
| 2.0813 | 1.8 | 2.0287 | 1.3 |

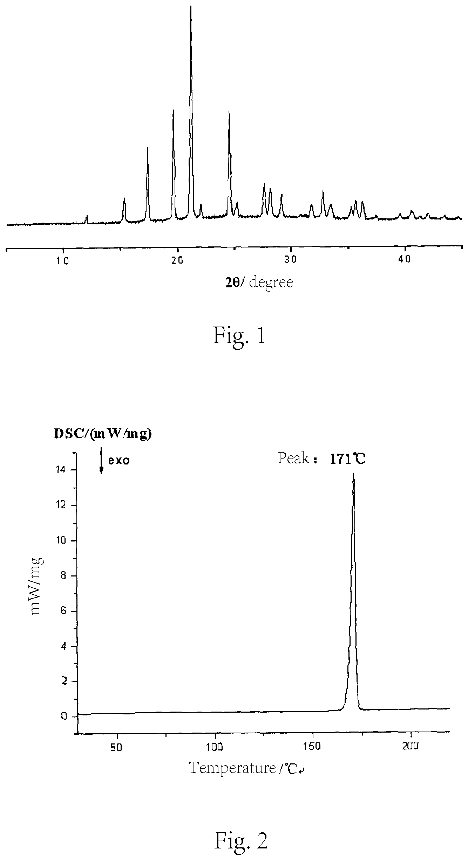

The crystal form I of the racemate of 4-hydroxy-2-oxo-1-pyrrolidine acetamide is characterized by a X-ray powder diffraction pattern shown in Figure 1.

The infrared spectrum produced by the crystal form I of the racemate of 4-hydroxy-2-oxo-1-pyrrolidine acetamide in the present application exhibits absorption peaks appearing at the following wave numbers:
3426 (cm⁻¹), 3330 (cm⁻¹), 3276 (cm⁻¹), 3235 (cm⁻¹), 2931 (cm⁻¹), 2876 (cm⁻¹), 1665 (cm⁻¹), 1593 (cm⁻¹), 1480 (cm⁻¹), 1452 (cm⁻¹),1428 (cm⁻¹), 1320 (cm⁻¹), 1272 (cm⁻¹), 1220 (cm⁻¹), 1078 (cm⁻¹),974 (cm⁻¹), 941 (cm⁻¹), 757 (cm⁻¹), 702 (cm⁻¹), 602 (cm⁻¹), 517(cm⁻¹) and 450 (cm⁻¹).

The preparation method of the crystal form I of the racemate of 4-hydroxy-2-oxo-1-pyrrolidine acetamide is characterized by the following steps:
(1). dissolving crude racemate of 4-hydroxy-2-oxo-1-pyrrolidine acetamide in isopropyl alcohol to reach supersaturation;
(2). heating and stirring for 7h at 38-42 °C to obtain a suspended sediment;
(3). filtering and drying to obtain crystals;
wherein said crude racemate of 4-hydroxy-2-oxo-1-pyrrolidine acetamide is synthesized by the steps (a) to (d) described below. In order to further improve the purity and yield of the crystal form I of the racemate of 4-hydroxy-2-oxo-1-pyrrolidine acetamide, the stirring is carried out at a temperature preferably of 40°C. In particular, the preparation method of the crystal form I of the racemate of 4-hydroxy-2-oxo-1-pyrrolidine acetamide is in accordance with the following procedure:
(1). dissolving crude racemate of 4-hydroxy-2-oxo-1-pyrrolidine acetamide in isopropyl alcohol to reach supersaturation and suspended matter precipites;
(2). heating and stirring the supersaturated solution with precipitated suspended matter at 40°C for 7h to obtain a suspended sediment;
(3). filtering and drying under vacuum for 24h to obtain crystalline powder.

The present invention has the following advantages:
The crystal form I of the racemate of 4-hydroxy-2-oxo-1-pyrrolidine acetamide obtained by the method of the present invention has a high purity which can reach 99.5% and is effective when used in drugs to treat corresponding diseases. The preparation method of the crystal form I the racemate of of 4-hydroxy-2-oxo-1-pyrrolidine acetamide is simple, with mild control conditions and low cost. The obtained crystal form of the racemate of 4-hydroxy-2-oxo-1-pyrrolidine acetamide has a high purity which can reach 99.5% and is in a desirable yield which can reach 90%. The preparation method is suitable for large-scale industrialization production.

### Brief Description of the Drawings

Figure 1 is an X-ray powder diffraction pattern of the crystal form I of the racemate of 4-hydroxy-2-oxo-1-pyrrolidine acetamide.
Figure 2 is a differential scanning calorimetry (DSC) thermogram of the crystal form I of the racemate of 4-hydroxy-2-oxo-1-pyrrolidine acetamide.
Figure 3 is a Raman spectrum of the crystal form I of the racemate of 4-hydroxy-2-oxo-1-pyrrolidine acetamide.
Figure 4 is an infrared (IR) spectrum of the crystal form I of the racemate of 4-hydroxy-2-oxo-1-pyrrolidine acetamide.
Figure 5 is a thermogravimetric analysis (TG) curve of the crystal form I of the racemate of 4-hydroxy-2-oxo-1-pyrrolidine acetamide.

### Detailed Description of the Preferred Embodiments

### Example 1 (reference)

50mg of crude racemate of 4-hydroxy-2-oxo-1-pyrrolidine acetamide was dissolved in absolute methanol to form a solution. The solution was stirred overnight and filtered. The resulting solution was left to stand in a dryer and the solvent was evaporated to obtain a colorless transparent crystal in 99.4% purity, and the yield was 82%.

The crude racemate of 4-hydroxy-2-oxo-1-pyrrolidine acetamide with a purity of 98.5% in the present invention is synthesized by the following steps:
(a) 139.6g of glycine ethyl ester hydrochloride was added to 1100mL of anhydrous ether, cooled to -6°C, and then 27.2g ammonia gas was introduced to form free glycine ethyl ester from glycine ethyl ester hydrochloride, wherein glycine ethyl ester hydrochloride: anhydrous ether: ammonia gas = 1mol: 1100mL: 1.6mol;
(b) To the above mixture 336mL of anhydrous alcohol and 67.2g of sodium bicarbonate were added, followed by the addition of 166.6g of ethyl 4-chloro-3-hydroxybutyrate in drops. The period for the addition in drops lasted for a time of 1.8h, then the reaction continued for 22h at a temperature of 72°C and pH=8;
(c) The above reaction solution was filtered, and the residue was fully washed with ethanol and the filtrate was concentrated. The concentrate was dissolved in water, and extracted by adding ethyl acetate 7 times to the weight of the filtrate. The aqueous phase was concentrated, and separated with column chromatography. Finally, aqueous ammonia of 23 wt % was added and then the reaction continued for 4h at 22 °C to obtain crude oxiracetam, wherein the amount of 23wt% aqueous ammonia is 13 times to the weight of the product separated by column chromatography;
   Wherein glycine ethyl ester: sodium bicarbonate: ethyl 4-chloro-3-hydroxybutyrate = 1: 0.8: 1 in moles, the amount of anhydrous alcohol is 5 times to the weight of sodium bicarbonate;
(d) The crude product obtained above was dissolved in water, treated by 732 # strong acid cation exchange resin, and then neutralized with 711 # strong base anion exchange resin; the solution was collected and concentrated; wherein the crude product: water = 1g: 0.5mL, the crude product: the strong acid cation exchange resin = 1g: 10mL.

### Example 2 (reference)

100mg of crude racemate of 4-hydroxy-2-oxo-1-pyrrolidine acetamide was dissolved in 95% alcohol, stirred overnight and filtered. The solution was left to stand in a dryer and the solvent was evaporated to obtain a colorless transparent crystal in 99.2% purity, and the yield is 85%.

### Example 3 (reference)

50mg of crude racemate of 4-hydroxy-2-oxo-1-pyrrolidine acetamide was dissolved in isopropyl alcohol to form a solution. The solution was stirred overnight and filtered. The solution after filtration was left to stand in a dryer and the solvent was evaporated to obtain a colorless transparent crystal in 99.2% purity, and the yield is 87%.

### Example 4 (reference)

50mg of crude racemate of 4-hydroxy-2-oxo-1-pyrrolidine acetamide was dissolved in absolute methanol to reach supersaturation, and much suspended matter precipited. The supersaturated solution was heated to 38°C and stirred for 10h. The suspended sediment was filtered and dried under vacuum for 24h. The precipitated powder is the crystal of racemate of 4-hydroxy-2-oxo-1-pyrrolidine acetamide in 99.5% purity, and the yield is 80%.

### Example 5 (reference)

50mg of crude racemate of 4-hydroxy-2-oxo-1-pyrrolidine acetamide was dissolved in anhydrous alcohol to reach supersaturation, and much suspended matter precipited. The supersaturated solution was heated and stirred at 40 °C overnight. The suspended sediment was filtered and dried under vacuum for 24h. The precipitated powder is the crystal of racemate of 4-hydroxy-2-oxo-1-pyrrolidine acetamide in 99.3% purity, and the yield is 80%.

### Example 6

50mg of crude racemate of 4-hydroxy-2-oxo-1-pyrrolidine acetamide was dissolved in isopropyl alcohol to reach supersaturation, and much suspended matter precipited, The supersaturated solution was heated and stirred at 40 °C for 7h. The suspended sediment was filtered and dried under vacuum for 24h. The precipitated powder is the crystal of racemate of 4-hydroxy-2-oxo-1-pyrrolidine acetamide in 99.5%purity, and the yield is 88%.

### Example 7 (reference)

50mg of crude racemate of 4-hydroxy-2-oxo-1-pyrrolidine acetamide was dissolved in propyl alcohol to reach supersaturation, and much suspended matter precipited. The supersaturated solution was heated and stirred at 42°C for 12h. The suspended sediment was filtered and dried under vacuum for 20h. The precipitated powder is the crystal of racemate of 4-hydroxy-2-oxo-1-pyrrolidine acetamide in 99.3% purity, and the yield is 80%.

### Example 8 (reference)

50mg of crude racemate of 4-hydroxy-2-oxo-1-pyrrolidine acetamide was dissolved in isopropyl alcohol to reach supersaturation, and much suspended matter precipited. The supersaturated solution was heated and stirred at 43°C for 11h. The suspended sediment was filtered and dried under vacuum for 18h. The precipitated powder is the crystal of racemate of 4-hydroxy-2-oxo-1-pyrrolidine acetamide in 99.2% purity, and the yield is 90%.

### Example 9 (reference)

50mg of crude racemate of 4-hydroxy-2-oxo-1-pyrrolidine acetamide was dissolved in n-butanol to reach supersaturation, and much suspended matter precipited. The supersaturated solution was heated and stirred at 45°C for 6h. The suspended sediment was filtered and dried under vacuum for 20h. The precipitated powder is the crystal of racemate of 4-hydroxy-2-oxo-1-pyrrolidine acetamide in 99.4% purity, and the yield is 80%.

### Example 10 determination of the crystal form I of the racemate of 4-hydroxy-2-oxo-1-pyrrolidine acetamide

The X-ray powder diffraction pattern of the crystal form I of the racemate of 4-hydroxy-2-oxo-1-pyrrolidine acetamide obtained in example 1 was determined using a Bruker D8 Advance diffractometer, and determination conditions is as follows: Cu Kα, 40kV, light source 40mV, step size 0.12°, scanning speed 10°/min, scanning range 5∼45°, room temperature. In the X-ray powder diffraction pattern of the crystal form I of the racemate of 4-hydroxy-2-oxo-1-pyrrolidine acetamide, diffraction peaks were appearing at the following 2θ diffraction angles: 12.011, 15.318, 17.407, 19.633, 21.228, 22.052, 24.577, 25.223, 27.647, 28.161, 29.109, 30.805, 31.276, 31.766, 32.77, 33.477, 35.252, 35.645, 36.236, 37.379, 39.56, 40.489, 41.256, 41.948, 43.443 and 44.628. The X-ray powder diffraction pattern is shown in Figure 1.

The X-ray powder diffraction pattern of the crystal form of racemate of 4-hydroxy-2-oxo-1-pyrrolidine acetamide in the present invention is expressed in terms of interplanar spacing d, Bragg angle (2θ) and relative intensity percentages I as shown below:

| 2θ / degree | d / Å | relative intensity I /% |
|---|---|---|
| 12. 011 | 7. 3623 | 3. 6 |
| 15. 318 | 5. 7794 | 11. 6 |
| 17. 407 | 5. 0905 | 34. 7 |
| 19. 633 | 4. 5179 | 51. 2 |
| 21. 228 | 4. 1820 | 100. 0 |
| 22. 052 | 4. 0275 | 5. 8 |
| 24. 577 | 3. 6192 | 49. 1 |
| 25. 223 | 3. 5278 | 6. 4 |
| 27. 647 | 3. 2238 | 15. 6 |
| 28. 161 | 3. 1661 | 13. 2 |
| 29. 109 | 3. 0652 | 10. 4 |
| 30. 805 | 2. 9002 | 1. 3 |
| 31. 276 | 2. 8576 | 1. 4 |
| 31. 766 | 2. 8146 | 5. 6 |
| 32. 770 | 2. 7306 | 12. 3 |
| 33. 477 | 2. 6746 | 5. 6 |
| 35. 252 | 2. 5438 | 5. 0 |
| 35. 645 | 2. 5167 | 8. 4 |
| 36. 236 | 2. 4770 | 7. 9 |
| 37. 379 | 2. 4038 | 1. 9 |
| 39. 560 | 2. 2762 | 2. 3 |
| 40. 489 | 2. 2261 | 3. 7 |
| 41. 256 | 2. 1865 | 1. 5 |
| 41.948 | 2.1520 | 2.6 |
| 43.443 | 2.0813 | 1.8 |
| 44.628 | 2.0287 | 1.3 |

Figure 1 is an X-ray powder diffraction pattern, which coincides with the X-ray powder diffraction pattern reported by CCDC, of the crystal form of the racemate of 4-hydroxy-2-oxo-1-pyrrolidine acetamide simulated from the crystal structure data.

The differential scanning calorimetry (DSC) thermogram of the crystal form of the racemate of 4-hydroxy-2-oxo-1-pyrrolidine acetamide is shown in Figure 2 where the thermal transition temperature is 171°C.

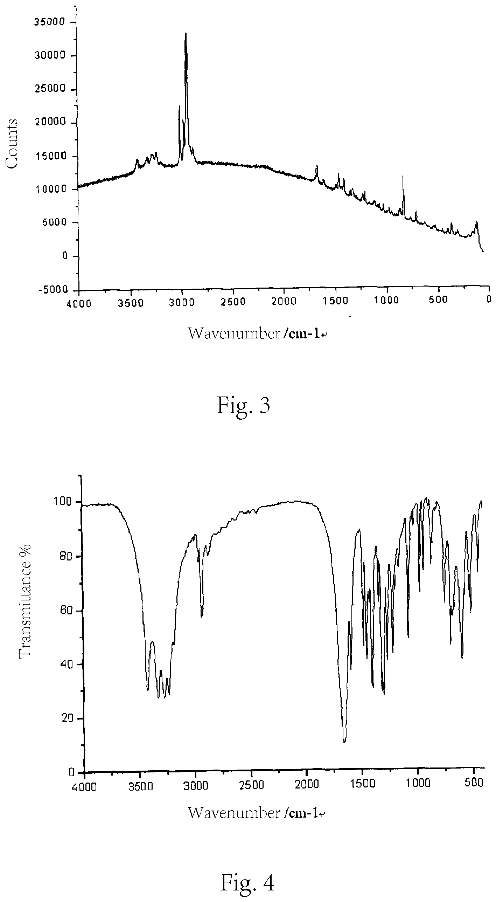

The Raman spectrum of the crystal form of the racemate of 4-hydroxy-2-oxo-1-pyrrolidine acetamide is shown in Figure 3.

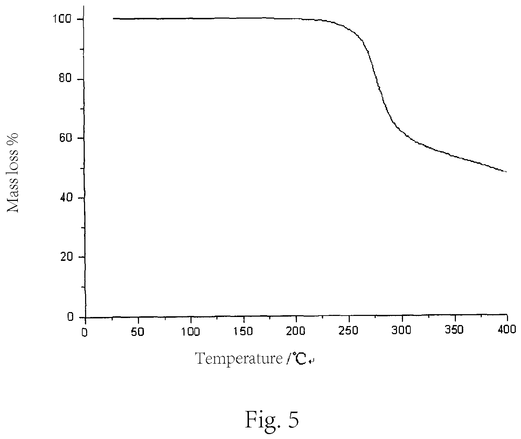

The thermogravimetric analysis curve of the crystal form of the racemate of 4-hydroxy-2-oxo-1-pyrrolidine acetamide is shown in Figure 5.

The infrared (IR) spectrum of the crystal form of the racemate of 4-hydroxy-2-oxo-1-pyrrolidine acetamide is shown in Figure 4, which exhibits absorption peaks appearing at the following wave numbers: 3426, 3330, 3276, 3235, 2931, 2876, 1665, 1593, 1480, 1452,1428, 1320, 1272, 1220, 1078,974, 941, 757, 702, 602, 517 and 450cm⁻¹.

## Claims

1. A preparation method of the crystal form I of the racemate of 4-hydroxy-2-oxo-1-pyrrolidine acetamide (oxiracetam) comprising the following steps:
(1). dissolving crude oxiracetam in isopropyl alcohol to reach supersaturation;
(2). heating and stirring for 7h at 38-42 °C to obtain a suspended sediment;
(3). filtering and drying to obtain a crystal;
wherein said crystal form I has diffraction peaks appearing at the following 2θ diffraction angles: 12.011, 15.318, 17.407, 19.633, 21.228, 22.052, 24.577, 25.223, 27.647, 28.161, 29.109, 30.805, 31.276, 31.766, 32.77, 33.477, 35.252, 35.645, 36.236, 37.379, 39.56, 40.489, 41.256, 41.948, 43.443 and 44.628;
wherein said crude oxiracetam is synthesized by the following steps:
(a) 139.6g of glycine ethyl ester hydrochloride was added to 1100mL of anhydrous ether, cooled to -6°C, and then 27.2g ammonia gas was introduced to form free glycine ethyl ester from glycine ethyl ester hydrochloride, wherein glycine ethyl ester hydrochloride: anhydrous ether: ammonia gas = 1mol:1100mL: 1.6mol;
(b) to the above mixture 336mL of anhydrous alcohol and 67.2g of sodium bicarbonate were added, followed by the addition of 166.6g of ethyl 4-chloro-3-hydroxybutyrate in drops; the period for the addition in drops lasted for a time of 1.8h, then the reaction continued for 22h at a temperature of 72 °C and pH=8;
(c) the above reaction solution was filtered, and the residue was fully washed with ethanol and the filtrate was concentrated; the concentrate was dissolved in water, and extracted by adding ethyl acetate 7 times to the weight of the filtrate; the aqueous phase was concentrated, and separated with column chromatography; finally, aqueous ammonia of 23 wt % was added and then the reaction continued for 4h at 22 °C to obtain crude oxiracetam, wherein the amount of 23wt% aqueous ammonia is 13 times to the weight of the product separated by column chromatography; wherein glycine ethyl ester: sodium bicarbonate: ethyl 4-chloro-3-hydroxybutyrate = 1: 0.8: 1 in moles, the amount of anhydrous alcohol is 5 times to the weight of sodium bicarbonate;
(d) the crude product obtained above was dissolved in water, treated by 732 # strong acid cation exchange resin, and then neutralized with 711 # strong base anion exchange resin; the solution was collected and concentrated; wherein the crude product: water = 1g: 0.5mL, the crude product: the strong acid cation exchange resin = 1g: 10mL.

2. The preparation method of the crystal form I of the racemate of 4-hydroxy-2-oxo-1-pyrrolidine acetamide according to claim 1, wherein the temperature of heating is 40 °C.

3. The preparation method of the crystal form I of the racemate of 4-hydroxy-2-oxo-1-pyrrolidine acetamide according to claim 1, wherein the filtering and drying of step (3) is under vacuum for 24h to obtain a crystalline powder.

## Patentansprüche

1. Herstellungsverfahren für die Kristallform I des Racemats von 4-Hydroxy-2-oxo-1-pyrrolidinacetamid (Oxiracetam), umfassend die folgenden Schritte:
(1) Lösen von rohem Oxiracetam in Isopropylalkohol, um eine Übersättigung zu erreichen;
(2) Erhitzen und Rühren für 7 h bei 38-42°C, um ein suspendiertes Sediment zu erhalten;
(3) Filtrieren und Trocknen, um einen Kristall zu erhalten;
wobei die Kristallform I Beugungspeaks aufweist, die bei den folgenden 2θ-Beugungswinkel auftreten: 12,011, 15,318, 17,407, 19,633, 21,228, 22,052, 24,577, 25,223, 27,647, 28,161, 29,109, 30,805, 31,276, 31,766, 32,77, 33,477, 35,252, 35,645, 36,236, 37,379, 39,56, 40,489, 41,256, 41,948, 43,443 und 44,628;
wobei das rohe Oxiracetam durch die folgenden Schritte synthetisiert wird:
(a) 139,6 g Glycinethylester-Hydrochlorid wurden zu 1100 mL wasserfreiem Ether gegeben, es wurde auf -6°C abgekühlt und dann wurden 27,2 g Ammoniakgas eingebracht, um freien Glycinethylester aus Glycinethylester-Hydrochlorid zu bilden, wobei Glycinethylester-Hydrochlorid: wasserfreiem Ether: Ammoniakgas = 1 Mol: 1100 mL: 1,6 Mol;
(b) zu dem obigen Gemisch wurden 336 mL wasserfreier Alkohol und 67,2 g Natriumhydrogencarbonat gegeben, gefolgt von der Zugabe von 166,6 g 4-Chlor-3-hydroxybuttersäureethylester in Tropfen; der Zeitraum für die Zugabe in Tropfen dauerte für eine Zeit von 1,8 h, dann dauerte die Reaktion für 22 h bei einer Temperatur von 72°C und pH = 8 fort;
(c) die obige Reaktionslösung wurde filtriert und der Rückstand wurde vollständig mit Ethanol gewaschen und das Filtrat wurde konzentriert; das Konzentrat wurde in Wasser gelöst und durch 7-maliges Zugeben von Ethylacetat zu dem Gewicht des Filtrats extrahiert; die wässrige Phase wurde konzentriert und mit Säulenchromatographie getrennt; schließlich wurden 23 Gew.-% wässriger Ammoniak zugegeben und dann dauerte die Reaktion für 4 h bei 22°C fort, um rohes Oxiracetam zu erhalten, wobei die Menge von 23 Gew.-% wässrigem Ammoniak das 13-fache des Gewichts des Produkts ist, das durch Säulenchromatographie getrennt wurde; wobei Glycinethylester: Natriumhydrogencarbonat: 4-Chlor-3-hydroxybuttersäureethylester = 1: 0,8: 1 in Mol, die Menge von wasserfreiem Alkohol das 5-fache des Gewichts von Natriumhydrogencarbonat ist;
(d) das oben erhaltene rohe Produkt wurde in Wasser gelöst, mit stark saurem Kationenaustauschharz Nr. 732 behandelt und dann mit stark basischem Anionenaustauschharz Nr. 711 neutralisiert; die Lösung wurde gesammelt und konzentriert; wobei das rohe Produkt: Wasser = 1 g: 0,5 mL, das rohe Produkt: das stark saure Kationenaustauschharz = 1 g: 10 mL.

2. Herstellungsverfahren für die Kristallform I des Racemats von 4-Hydroxy-2-oxo-1-pyrrolidinacetamid nach Anspruch 1, wobei die Temperatur des Erhitzens 40°C ist.

3. Herstellungsverfahren für die Kristallform I des Racemats von 4-Hydroxy-2-oxo-1-pyrrolidinacetamid nach Anspruch 1, wobei das Filtrieren und Trocknen von Schritt (3) unter Vakuum für 24 h ist, um ein kristallines Pulver zu erhalten.

## Revendications

1. Procédé de préparation de la forme cristalline I du racémate de 4-hydroxy-2-oxo-1-pyrrolidine-acétamide (oxiracétam) comprenant les étapes suivantes :
(1) dissolution d'oxiracétam brut dans de l'alcool isopropylique pour atteindre la supersaturation ;
(2) chauffage et agitation pendant 7 h à 38-42 °C pour obtenir un sédiment en suspension ;
(3) filtration et séchage pour obtenir un cristal ;
dans lequel ladite forme cristalline I présente des pics de diffraction apparaissant aux angles de diffraction 2θ suivants : 12,011, 15,318, 17,407, 19,633, 21,228, 22,052, 24,577, 25,223, 27,647, 28,161, 29,109, 30,805, 31,276, 31,766, 32,77, 33,477, 35,252, 35,645, 36,236, 37,379, 39,56, 40,489, 41,256, 41,948, 43,443 et 44,628 ;
dans lequel ledit oxiracétam brut est synthétisé par les étapes suivantes :
(a) 139,6 g de chlorhydrate d'ester éthylique de glycine ont été ajoutés à 1100 ml d'éther anhydre, refroidi à -6 °C, puis 27,2 g de gaz d'ammoniac ont été introduits pour former de l'ester éthylique de glycine libre à partir de chlorhydrate d'ester éthylique de glycine, dans lequel le chlorhydrate d'ester éthylique de glycine : éther anhydre : gaz d'ammoniac = 1 mol : 1100 ml : 1,6 mol ;
(b) au mélange ci-dessus 336 ml d'alcool anhydre et 67,2 g de bicarbonate de sodium ont été ajoutés, ce qui a été suivi de l'ajout de 166,6 g de 4-chloro-3-hydroxybutyrate d'éthyle goutte à goutte ; la période pour l'ajout goutte à goutte a duré un temps de 1,8h, puis la réaction s'est poursuivie pendant 22 h à une température de 72 °C et au pH = 8 ;
(c) la solution réactionnelle ci-dessus a été filtrée, et le résidu a été entièrement lavé à l'éthanol et le filtrat a été concentré ; le concentré a été dissous dans l'eau, et extrait par ajout d'acétate d'éthyle à raison de 7 fois le poids du filtrat ; la phase aqueuse a été concentrée, et séparée par chromatographie sur colonne ; enfin, de l'ammoniaque aqueuse à 23 % en poids a été ajoutée puis la réaction s'est poursuivie pendant 4 h à 22 °C pour obtenir de l'oxiracétam brut, dans lequel la quantité d'ammoniaque aqueuse à 23 % en poids est de 13 fois le poids du produit séparé par chromatographie sur colonne ; dans lequel l'ester éthylique de glycine : bicarbonate de sodium : 4-chloro-3-hydroxybutyrate d'éthyle = 1 : 0,8 : 1 en moles, la quantité d'alcool anhydre est de 5 fois le poids du bicarbonate de sodium ;
(d) le produit brut obtenu ci-dessus a été dissous dans l'eau, traité par la résine échangeuse de cations fortement acide n° 732, puis neutralisé avec la résine échangeuse d'anions fortement basique n° 711 ; la solution a été recueillie et concentrée ; dans lequel le produit brut : eau = 1 g : 0,5 ml, le produit brut : la résine échangeuse de cations fortement acide = 1 g : 10 ml.

2. Procédé de préparation de la forme cristalline I du racémate de 4-hydroxy-2-oxo-1-pyrrolidine-acétamide selon la revendication 1, dans lequel la température de chauffage est de 40 °C.

3. Procédé de préparation de la forme cristalline I du racémate de 4-hydroxy-2-oxo-1-pyrrolidine-acétamide selon la revendication 1, dans lequel la filtration et le séchage de l'étape (3) sont sous vide pendant 24 h pour obtenir une poudre cristalline.
